# EUROPEAN PATENT APPLICATION

(11) **EP 4 238 524 A1**
(43) Date of publication of application: **06.09.2023**
(21) Application number: 21886144.1
(22) Date of filing: 25.10.2021
(51) Int. Cl.: A61B 90/00, A61B 1/00, A61B 1/04, A61B 17/04, A61B 17/34

(54) **SURGERY ASSISTANCE TOOL AND SURGERY ASSISTANCE SYSTEM**

(30) Priority: 29.10.2020 JP 2020181155
(71) Applicant: National University Corporation Tokai National Higher Education and Research System, Nagoya-shi, Aichi 464-8601 (JP); Nagoya Denki Educational Foundation, Aichi 464-8540 (JP)
(72) Inventor: NAKAMURA, Shota, Nagoya-shi, Aichi 464-8601 (JP); YOSHIKAWA, Toyofumi, Nagoya-shi, Aichi 464-8601 (JP); KITASAKA, Takayuki, Toyota-shi, Aichi 470-0392 (JP); HAYASHI, Yuichiro, Nagoya-shi, Aichi 464-8601 (JP); MORI, Kensaku, Nagoya-shi, Aichi 464-8601 (JP)
(74) Representative: Heyerhoff Geiger & Partner Patentanwälte PartGmbB
(86) International application number: PCT/JP2021/039295
(87) International publication number: WO 2022/092026

(57) **Abstract**

An object is to provide a surgical assistance device and a surgery support system that can ensure a sufficient field of view during an arthroscopic surgery. The object can be achieved by a surgical assistance device for capturing an image inside a body cavity. The surgical assistance device includes: N image capturing units; holding parts, each of the holding parts holding each of the image capturing units; and a base member, and N is an integer that is greater than or equal to three. When N virtual lines each passing through each of the holding parts holding the N image capturing units are defined as reference lines for arrangement when the N virtual lines are substantially parallel to each other and substantially perpendicular to a virtual plane formed by the base member or the holding parts, each of the image capturing units is arranged so as to be oriented to outside at an angle that is greater than or equal to 0 degree and less than or equal to 10 degrees relative to each of the reference lines passing through each of the holding parts.

## Description

### [Technical Field]

The disclosure in the present application relates to a surgical assistance device and a surgery support system.

### [Background Art]

In the field of surgical operations, arthroscopic surgeries such as laparoscopic surgeries or thoracoscopic surgeries have rapidly become prevalent in place of surgeries under direct view such as conventional laparotomy surgeries or thoracotomy serveries. The arthroscopic surgeries have various advantages in terms of esthetic outcome, low invasiveness, or the like.

In arthroscopic surgeries, unlike laparotomy surgeries or thoracotomy serveries, the operator is unable to directly view the affected part. Thus, there is a known example in which a plurality of trocars having cameras are inserted in the body, images obtained from the cameras are synthesized based on camera positions estimated by position sensors, and the operator operates a surgical instrument such as a forceps while viewing a monitor displaying the synthesized image (see Patent Literature 1).

### [Citation List]

### [Patent Literature]

Patent Literature 1: Japanese Patent No. 5975504

### [Summary of Invention]

### [Technical Problem]

Even with today's advanced medical technologies, deaths due to intraoperative vascular injuries have been reported. According to a questionnaire survey by the Japanese Society for Endoscopic Surgery, many surgeons cite "insufficient field of view" as one of the causes of vascular injuries. It is thus desired to ensure a wide field of view required for proceeding a safe surgery (hereafter, which may be simply referred to as "field of view") in the arthroscopic surgery. In the art disclosed in Patent Literature 1, however, each camera is arranged at the tip of the trocar inserted in the body. Therefore, although it is possible to capture an image of a part of the body inside the camera, it is not possible to capture an image of a part of the body outside the camera, and thus, there is a problem of inability of panoramically viewing the field of view.

Further, in the art disclosed in Patent Literature 1, a plurality of trocars each having a camera arranged at the tip are inserted in the body. That is, the positional relationship among the cameras arranged to the trocars varies for each surgery or during the surgery. Thus, in the art disclosed in Patent Literature 1, the trocars have position markers, and it is required to detect the position markers by position sensors and synthesize images obtained based on estimated positions of the cameras. Thus, there is a problem of complex image synthesis.

The disclosure in the present application has been made to solve the above problems and newly found through an intensive study that, by arranging a surgical assistance device having three or more image capturing units arranged to a base member at an incision site during a surgery, it is possible to ensure a sufficient field of view without requiring position markers.

That is, an object of the disclosure in the present application is to provide a surgical assistance device and a surgery support system that can ensure a sufficient field of view during an arthroscopic surgery.

### [Solution to Problem]

The disclosure in the present application relates to a surgical assistance device and a surgery support system illustrated below.
(1) A surgical assistance device for capturing an image inside a body cavity, the surgical assistance device comprising:
   N image capturing units;
   holding parts, each of the holding parts holding each of the image capturing units; and
   a base member,
   wherein N is an integer that is greater than or equal to three, and
   wherein when N virtual lines each passing through each of the holding parts holding the N image capturing units are defined as reference lines for arrangement when the N virtual lines are
   substantially parallel to each other, and
   substantially perpendicular to a virtual plane formed by the base member or the holding parts,
   each of the image capturing units is arranged so as to be oriented to outside at an angle that is greater than or equal to 0 degree and less than or equal to 10 degrees relative to each of the reference lines passing through each of the holding parts.
(2) The surgical assistance device according to (1) above, wherein the holding parts are formed as separate components from the base member.
(3) The surgical assistance device according to (1) or (2) above, wherein the angle of at least one of the N image capturing units is variable.
(4) The surgical assistance device according to (2) or (3) above, wherein the holding parts formed as the separate components from the base member are formed rotatably relative to the base member.
(5) The surgical assistance device according to any one of (1) to (4) above, wherein the base member is formed of a flexible material.
(6) The surgical assistance device according to any one of (1) to (5) above, wherein each of the image capturing units is arranged so as to be oriented to outside of the base member at an angle that is greater than or equal to 5 degrees and less than or equal to 10 degrees relative to each of the reference lines passing through each of the holding parts.
(7) A surgery support system comprising:
   the surgical assistance device according to any one of (1) to (6) above; and
   an image processing unit that synthesizes images obtained from the image capturing units.
(8) The surgery support system according to (7) above further comprising a display unit that displays an image processed by the image processing unit.
(9) A program used for the surgery support system according to (8) above.
(10) The program according to (9) above, wherein when any of the image capturing units captures a surgical instrument during a surgery, the program performs image processing so that the surgical instrument is not displayed on the display unit based on
   an image captured by another of the image capturing units, or
   an image inside a body cavity captured before image capturing of the surgical instrument is detected.

### [Advantageous Effects]

The surgical assistance device and the surgery support system disclosed in the present application can be suitably used for arthroscopic surgeries.

### [Brief Description of Drawings]

[FIG. 1] FIG. 1A is a schematic top view of a surgical assistance device 1a, FIG. 1B is a sectional view taken along X-X' of FIG. 1A, and FIG. 1C is a diagram with removal of an image capturing unit 2 from FIG. 1B.
[FIG. 2] FIG. 2A is a schematic top view of the surgical assistance device 1a, and FIG. 2B is a front view of FIG. 2A.
[FIG. 3] FIG. 3A and FIG. 3B are enlarged views of a portion surrounded by a circle of FIG. 2B.
[FIG. 4] FIG. 4 is a view when FIG. 3A is viewed from the top, which is a diagram illustrating a direction dα in which an angle α is formed.
[FIG. 5] FIG. 5A and FIG. 5B are diagrams illustrating positions at which image capturing units 2 are arranged.
[FIG. 6] FIG. 6A is a schematic top view of a surgical assistance device 1b, FIG. 6B is a sectional view taken along X-X' of FIG. 6A, and FIG. 6C is an enlarged view of a portion surrounded by a circle of FIG. 6A with removal of an image capturing unit 2.
[FIG. 7] FIG. 7A is a schematic top view of a surgical assistance device 1c, FIG. 7B is a sectional view taken along X-X' of FIG. 7A, and FIG. 7C is a diagram with removal of an image capturing unit 2 from FIG. 7B.
[FIG. 8] FIG. 8A is a schematic top view of a surgical assistance device 1d, FIG. 8B is a sectional view taken along X-X' of FIG. 8A, and FIG. 8C is a diagram after a holding part 3a of FIG. 8B has been rotated in an R1 direction.
[FIG. 9] FIG. 9 is a schematic top view of a surgical assistance device 1e.
[FIG. 10] FIG. 10 is a schematic diagram illustrating an overview of a surgery support system 10.
[FIG. 11] FIG. 11A is a photograph substitute for a drawing, which is a photograph of the surgical assistance device produced in Example 1. FIG. 11B is a photograph substitute for a drawing, which is a photograph when the surgical assistance device is inserted in a mock human body
[FIG. 12] FIG. 12A to FIG. 12C are diagrams illustrating the arrangement of the surgical assistance device in an observation experiment inside a body cavity using a mock human body.
[FIG. 13] FIG. 13A to FIG. 13C are photographs substitute for drawings, which are synthesized images obtained in Example 3.
[FIG. 14] FIG. 14A to FIG. 14C are photographs substitute for drawings, which illustrate synthesized images obtained in Example 4.
[FIG. 15] FIG. 15A to FIG. 15C are photographs substitute for drawings, which illustrate synthesized images obtained in Example 5.
[FIG. 16] FIG. 16A to FIG. 16C are photographs substitute for drawings, which illustrate synthesized images obtained in Example 6.
[FIG. 17] FIG. 17 includes photographs substitute for drawings, which illustrate synthesized images obtained in Example 7.
[FIG. 18] FIG. 18D1 is a diagram illustrating the arrangement of a surgical assistance device produced in Comparative example 1 to a mock human body. FIG. 18D2 is a photograph substitute for a drawing, which is a synthesized image obtained in Comparative example 1.
FIG. 18A is a photograph substitute for a drawing, which is the same as FIG. 14A.
[FIG. 19] FIG. 19E and FIG. 19F illustrate the arrangement of a surgical assistance device produced in Example 8 to a mock human body and obtained synthesized images.

### [Description of Embodiments]

A surgical assistance device and a surgery support system disclosed in the present application will be described below in detail. Note that the position, size, range, or the like of each component illustrated in the drawings may not represent the actual position, size, range, or the like for easier understanding. Thus, the disclosure of the present application is not necessarily limited to the position, the size, the range, or the like disclosed in the drawings.

Further, in the present specification, the following interpretation will be applied for expression of numerical values, abbreviations, or the like.
(1) A numerical range represented by using "to" means a range including numerical values written before and after "to" as the lower limit value and the upper limit value.
(2) A numerical value, a numerical range, and a qualitative expression (for example, expression of "identical", "the same", or the like) indicate a numerical value, a numerical range, and a nature including an error generally tolerated in the field of the art of interest.
(3) When "substantially XX" is referred to, this includes a state recognized as approximately XX in addition to the exact XX. For example, when substantially parallel is referred to, this means that a slight shift is tolerated in addition to a state of being exactly parallel.

### [Embodiment of Surgical Assistance Device]

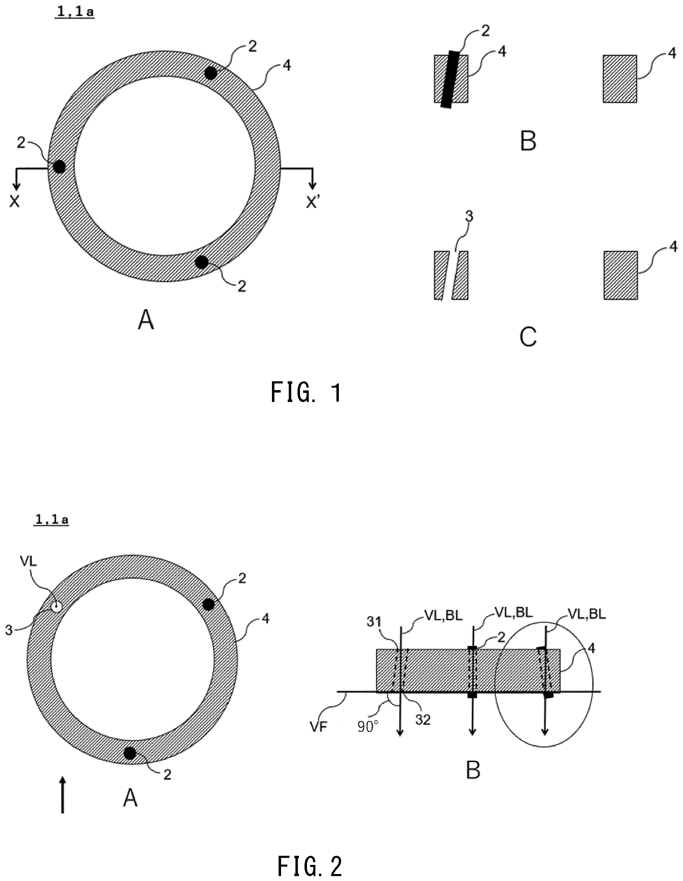

A surgical assistance device 1a according to an embodiment will be described with reference to FIG. 1. FIG. 1A is a schematic top view of the surgical assistance device 1a, FIG. 1B is a sectional view taken along X-X' of FIG. 1A, and FIG. 1C is a diagram with removal of an image capturing unit 2 from FIG. 1B.

The surgical assistance device 1a includes image capturing units 2, holding parts 3 that hold the image capturing units 2, and a base member 4. In the example illustrated in FIG. 1A to FIG. 1C, the base member 4 includes the holding parts 3.

Each image capturing unit 2 is not particularly limited as long as it can capture an image inside a body cavity. For example, a CCD image sensor, a CMOS image sensor, Foveon X3, an organic thin-film image capturing element, or the like may be employed. Note that the image capturing range of the image capturing unit 2 is not particularly limited. While it is conceivable to use a wide-angle camera to capture an image inside a body cavity by a single image capturing unit 2, in such a case, the edge of an image may be blurred. Further, there may be a part unable to be captured due to a surgical instrument, an organ, or the like (a part hidden behind the same). In contrast, since the surgical assistance device 1a is equipped with three or more image capturing units 2, it is possible to ensure a sufficient field of view and reduce a hidden part even with use of the sensor or the like described above that are commonly, commercially available.

In the example illustrated in FIG. 1A, three image capturing units 2 are provided to the base member 4. As will be illustrated in Examples and Comparative examples described later, although a sufficient field of view was not ensured when the number of image capturing units 2 is two, a sufficient field of view was ensured when the number thereof was three or greater. Therefore, the number (N) of image capturing units 2 is not particularly limited as long as it is an integer of three or greater and may be, for example, four or greater, five or greater, six or greater, or the like. On the other hand, although there is no limitation on the upper limit of the number of image capturing units 2 in terms of ensuring a sufficient field of view, a larger number of image capturing units 2 will make the process in image synthesis complex and also increase the cost. Therefore, the upper limit of the number (N) of the image capturing units 2 can be considered taking the cost, the usability (processing speed) of image processing, or the like into account and may be, for example, 20 or less, 15 or less, 10 or less, 8 or less, or the like.

The holding parts 3 are provided in the base member 4 for holding the image capturing units 2. Although each holding part 3 is formed so as to penetrate through the base member 4 in the example illustrated in FIG. 1A to FIG. 1C, the shape or the arranged place of the holding part 3 is not particularly limited as long as it can hold the image capturing unit 2 at a predetermined angle, as described later.

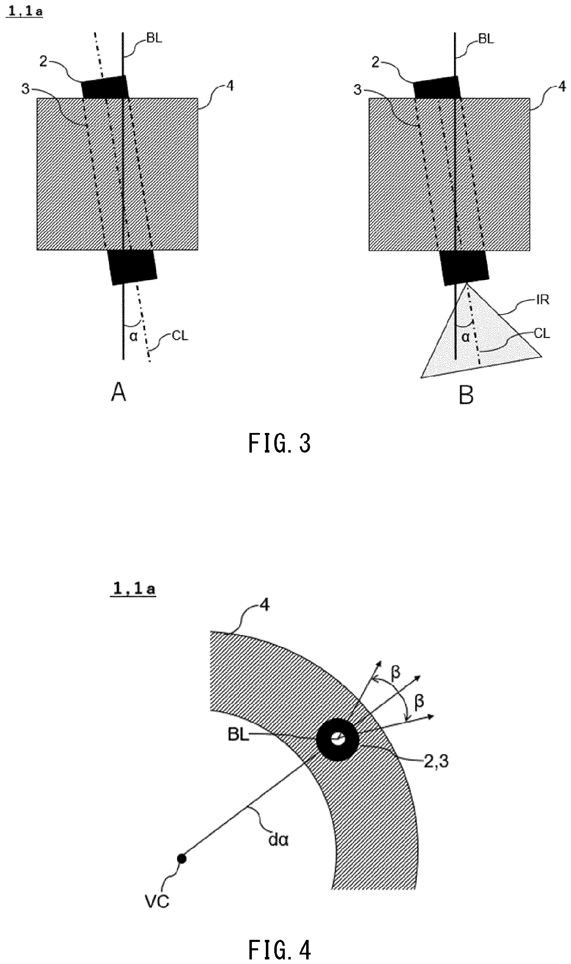

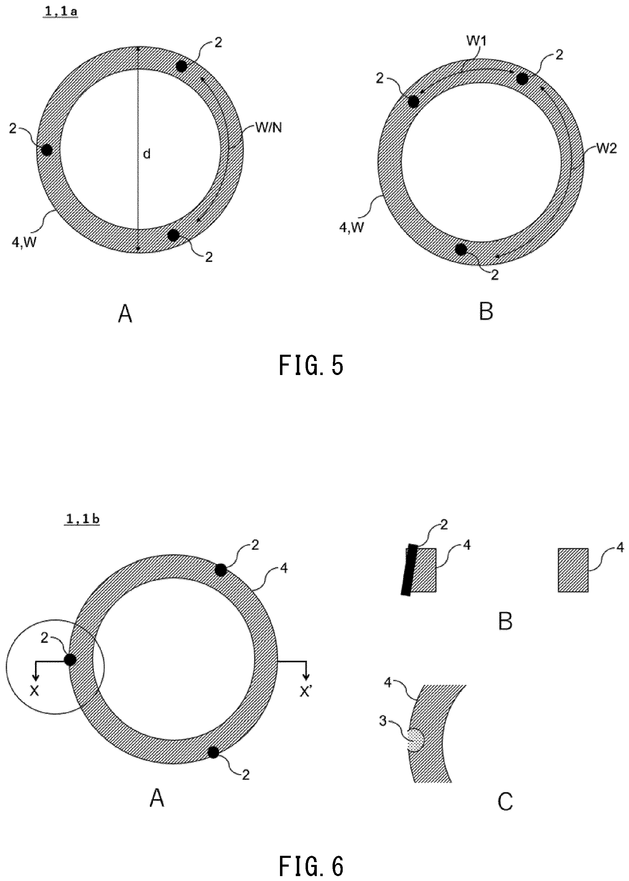

The angle of the image capturing unit 2 held by the holding part 3 will be described with reference to FIG. 2 to FIG. 5. FIG. 2A is a schematic top view of the surgical assistance device 1a (depiction of the top left image capturing unit 2 is omitted for the purpose of illustration), and FIG. 2B is a front view of FIG. 2A (a diagram viewed from the arrow direction of FIG. 2A). FIG. 3A and FIG. 3B are enlarged views of a portion surrounded by a circle of FIG. 2B. FIG. 4 is a view when FIG. 3A is viewed from the top, which is a diagram illustrating a direction dα in which an angle α is formed. FIG. 5A and FIG. 5B are diagrams illustrating positions at which image capturing units 2 are arranged.

First, each virtual line VL is defined as a reference line BL, for arrangement where virtual lines VL passing through the holding parts 3 are substantially parallel to each other and substantially perpendicular to the virtual plane VF formed by the base member 4 or the holding parts 3. Each of the image capturing units 2 is arranged so as to be oriented to outside of the base member 4 at an angle α that is greater than or equal to 0 degree and less than or equal to 10 degrees relative to the reference line BL passing through each of the holding parts 3.

The place through which the holding part 3 passes is not particularly limited as long as the virtual line VL passes through the holding part 3. If the virtual line VL satisfies the condition to be the reference line described above regardless of which holding part 3 the virtual line VL passes through, the angle relative to the image capturing unit 2 held by the holding part 3 can be defined.

Although the lower end side of the base member 4 is defined as the virtual plane VF in the example illustrated in FIG. 2B, the virtual plane VF may be on the upper end side of the base member 4. Alternatively, a plane including upper ends 31 of the holding parts 3 may be the virtual plane, or a plane including lower ends 32 of the holding parts 3 may be the virtual plane. Note that, when there is unevenness on the lower end side (or the upper end side) of the base member 4, the lower end side (or the upper end side) is not a completely flat plane in some cases. In such a case, a plane derived from the shape of the lower end side (or the upper end side) of the base member 4 by a least-squares method (least-squares plane) can be the virtual plane VF. When the number of holding parts 3 is four or greater and the upper ends 31 (or the lower ends 32) of respective holding parts 3 are not on the same plane, the least-squares plane can be the virtual plane VF.

The image capturing unit 2 is held by the holding part 3 at the angle α that is greater than or equal to 0 degree and less than or equal to 10 degrees relative to the reference line BL passing through the holding part 3. Note that, in the example illustrated in FIG. 3A, the angle α is defined by the center line CL passing through the center of the image capturing unit 2 and the reference line BL. Alternatively, the center of the image capturing range (IR) of the image capturing unit 2 may be the center line CL. The surgical assistance device 1a is used so as to be fitted to a portion incised by a scalpel during a surgery. In the example illustrated in FIG. 2B, the surgical assistance device 1a is fitted in the arrow direction on the reference line BL. Therefore, it can be said that the reference line BL is the insertion direction of the surgical assistance device 1a.

The angle α of the image capturing unit 2 relative to the reference line BL is set so that the image capturing unit 2 is oriented to outside of the base member 4 at the angle α that is greater than or equal to 0 degree and less than or equal to 10 degrees. As will be illustrated in Examples described later, the arrangement of the image capturing units 2 relative to ribs is adjusted, and thereby a sufficient field of view can be ensured even when the angle α is 0 degree. However, it is desirable that the surgical assistance device 1a can be used without taking into account of the arrangement of the image capturing unit 2 relative to the rib. In such a case, the angle α is desirably greater than 0 degree and can be suitably adjusted to be, for example, 0.5 degrees or greater, 1 degree or greater, 1.5 degrees or greater, 2 degrees or greater, 2.5 degrees or greater, 3 degrees or greater, 3.5 degrees or greater, 4 degrees or greater, 4.5 degrees or greater, 5 degrees or greater, or the like. In contrast, if the angle α of the image capturing unit 2 is excessively large, in other words, if the image capturing unit 2 is excessively oriented to outside of the base member 4, it will be difficult to obtain an image directed to the center of the base member 4. Therefore, the angle α can be suitably adjusted to be 12 degrees or less, 11.5 degrees or less, 11 degrees or less, 10.5 degrees or less, 10 degrees or less, 9.5 degrees or less, 9 degrees or less, 8.5 degrees or less, 8 degrees or less, or the like. Note that angles α of N image capturing units 2 may be the same or may be different, respectively.

The direction of the angle α of the image capturing unit 2 relative to the reference line BL will be described with reference to FIG. 2 to FIG. 4. FIG. 4 is a diagram illustrating the direction dα in which the angle α is formed. The arrangement of the image capturing unit 2 is not particularly limited as long as the direction connecting the reference line BL to the center line CL of the image capturing unit 2 is oriented to outside of the base member 4 (in a direction away from the base member 4). When the base member 4 has a hollow cylindrical shape as illustrated in FIG. 4, the direction dα in which the angle α is formed may be, for example, a direction connecting the virtual center VC of the substantially cylindrical shape to the reference line BL (or the image capturing unit 2 or the holding part 3). When the top view of the base member 4 is an ellipse, the virtual center VC can be the intersecting point of the longer axis and the shorter axis. Further, when the top view of the base member 4 is a shape other than a circle or an ellipse, the virtual center VC can be the centroid, for example. Note that FIG. 4 illustrates one example of the direction dα in which the angle α is formed, the direction may be shifted by a predetermined angle from dα illustrated in FIG. 4 as long as it is within a range that does not affect image capturing by the image capturing unit 2. For example, when the shift from dα illustrated in FIG. 4 is defined as an angle β, the angle β may be 0 degree or greater and 15 degrees or less, 10 degrees or less, 7.5 degrees or less, 5 degrees or less, 2.5 degrees or less, or the like. Note that angles β of N image capturing units 2 may be the same or may be different, respectively.

Next, positions at which N image capturing units 2 are arranged will be described with reference to FIG. 5. In the example illustrated in FIG. 5A, N image capturing units 2, where N = 3, are arranged at substantially equal intervals in the base member 4. Alternatively, the image capturing units 2 may not be arranged at equal intervals in the base member 4 as illustrated in FIG. 5B as long as a sufficient field of view can be ensured. In the example illustrated in FIG. 5A, each length between any image capturing unit 2 and its adjacent image capturing unit 2 is W/N, where the outer circumferential length of the base member 4 is W and when N image capturing units 2 are arranged at the equal intervals. As illustrated in FIG. 5B, when the image capturing units 2 are arranged at any positions, the shortest length between adjacent image capturing units 2 is defined as W1, and the longest length between adjacent image capturing units 2 is defined as W2. When W/N is 1, W1 can be 0.7 or greater, 0.75 or greater, 0.8 or greater, 0.85 or greater, or 0.9 or greater. Further, W2 can be 1.3 or less, 1.25 or less, 1.2 or less, 1.15 or less, or 1.1 or less. The same applies to a case where the base member 4 is an ellipse.

The base member 4 is not particularly limited in its material or the like as long as the holding parts 3 can be formed therein to hold the image capturing units 2. For example, an inflexible material that does not cause a change in the shape of the base member 4 may be used. Alternatively, the base member 4 may be formed of a flexible material that causes a change in the shape when the surgical assistance device 1a is inserted in an incised part. For example, a known medical material can be used for the inflexible material and the flexible material. The inflexible material may be, for example, a medical plastic such as polysulfone, polyvinylidene fluoride, polycarbonate, polypropylene, or the like; or a metal such as titanium, stainless, or the like. Further, the flexible material may be a medical plastic such as silicon, polyvinyl chloride, or the like.

When the surgical assistance device 1a is used for a thoracoscopic surgery, the surgical assistance device 1a is arranged between ribs. Thus, as illustrated in FIG. 5A, when the top view of the surgical assistance device 1a is substantially circular, the diameter d of the base member 4 may be about 30 mm to 150 mm, preferably, about 40 mm to 100 mm. Further, when the surgical assistance device 1a is used for laparoscopic surgeries, the diameter of the base member 4 may be about 30 mm to 150 mm, preferably, about 40 mm to 100 mm and may be larger than the numerical values noted above because of the absence of ribs. Note that, when the base member 4 is a shape other than a circle, the shortest distance among distances obtained when the base member 4 is pinched by parallel lines of vernier calipers or the like can be defined as the diameter. On the other hand, if the base member 4 has an excessively thin, long shape, the spacing between image capturing units may be excessively large. Therefore, when the shortest distance among distances obtained when the base member 4 is pinched by parallel lines of vernier calipers or the like is defined as 1, the longest distance may be 2 or less, 1.9 or less, 1.8 or less, 1.7 or less, 1.6 or less, 1.5 or less, or the like.

The surgical assistance device 1a according to the embodiment can be produced by using a 3D printer or the like to form the base member 4 equipped with the holding parts 3 and then inserting the image capturing units 2 in the holding parts 3.

Once a surgery support system is constructed with the surgical assistance device 1a according to the embodiment, it is possible to obtain an image panoramically viewing inside of a body cavity from a position where the surgical assistance device 1a is arranged. Therefore, a field of view required in a surgery can be ensured. Further, in operating a surgical instrument or the like, even when there is a portion where one image capturing unit is unable to capture an image due to the surgical instrument, it is possible to capture an image inside a body cavity by using another image capturing unit. Furthermore, because the relative positional relationship among the image capturing units 2 does not change, it is possible to easily synthesize an image without requiring use of position sensors or the like.

### [Modified Example to Embodiment of Surgical Assistance Device]

Next, modified examples to the embodiment of the surgical assistance device 1a will be described. The surgical assistance device 1a is the same as the surgical assistance device according to the modified examples except for those described below. Accordingly, for the modified examples, duplicated description for the features that have already been described in the embodiment of the surgical assistance device 1a will be omitted. It is thus apparent that, even though not explicitly described in the modified examples, the features that have already been described in the embodiment of the surgical assistance device 1a can be employed.

### [Modified Example 1 of Holding Part 3]

FIG. 6 illustrates modified example 1 of the holding part 3. FIG. 6A is a schematic top view of a surgical assistance device 1b, FIG. 6B is a sectional view taken along X-X' of FIG. 6A, and FIG. 6C is an enlarged view of a portion surrounded by a circle of FIG. 6A with removal of the image capturing unit 2. Each holding part 3 of the surgical assistance device 1a illustrated in FIG. 6A to FIG. 6C is formed as a cutout in the outer circumferential face of the base member 4. In the surgical assistance device 1a, since the holding part 3 is formed as a through hole penetrating the base member 4, it is required to insert the image capturing unit 2 from one side of the through hole. In contrast, in the modified example illustrated in FIG. 6A to FIG. 6C, it is possible to fit the image capturing unit 2 from the outer circumferential face of the base member 4. Therefore, in addition to the advantageous effects achieved by the surgical assistance device 1a, an advantageous effect of easier attachment of the image capturing unit 2 is achieved.

### [Modified Example 2 of Holding Part 3]

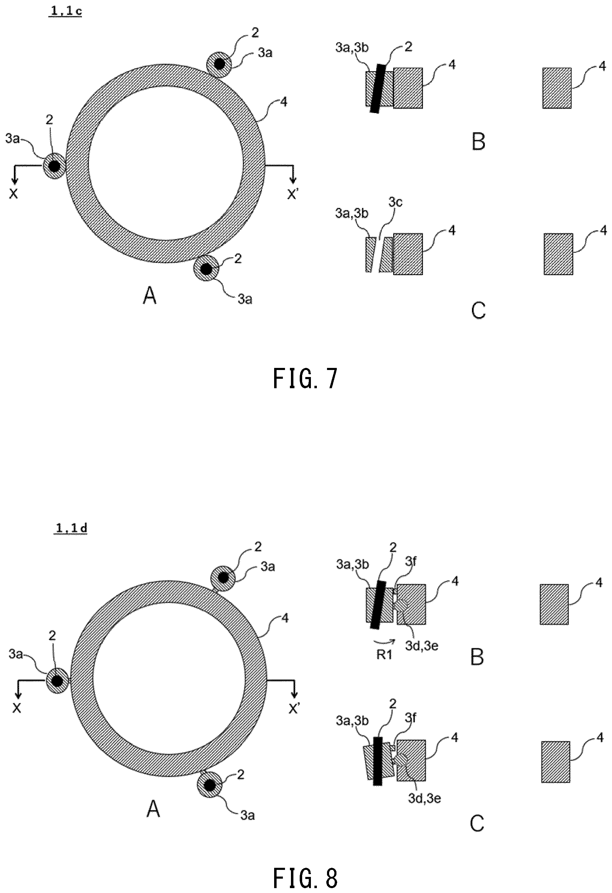

Fig. 7 illustrates modified example 2 of the holding part 3. FIG. 7A is a schematic top view of a surgical assistance device 1c, FIG. 7B is a sectional view taken along X-X' of FIG. 7A, and FIG. 7C is a diagram with removal of the image capturing unit 2 from FIG. 7B. The holding part 3a of the surgical assistance device 1c illustrated in FIG. 7A to FIG. 7C is formed as a separate component from the base member 4. The holding part 3 of the surgical assistance device 1a is a through hole penetrating the base member 4. In contrast, the holding part 3a according to modified example 2 differs from the holding part 3 of the surgical assistance device 1a in that a through hole 3c that holds the image capturing unit 2 is formed in a holding part base member 3b.

The same material as that for the base member 4 described above can be used for the holding part base member 3b, and the material forming the base member 4 and the material forming the holding part base member 3b are the same or may be different from each other. Further, the angle of the image capturing unit 2 held by the through hole 3c is the same as that of the surgical assistance device 1a. Further, although the through hole 3c is formed so as to penetrate the holding part base member 3b in the example illustrated in FIG. 7, a cutout may be formed in the outer circumferential face of the holding part base member 3b, and the image capturing unit 2 may be fitted from the outer circumferential face of the holding part base member 3b, as illustrated in modified example 1. Further, in the example illustrated FIG. 7A to FIG. 7C, the holding part 3a and the base member 4 are directly connected to each other. Alternatively, the holding part 3a and the base member 4 may be connected via a coupling part (not illustrated). Note that, although the holding part 3a and the base member 4 are formed as separate components, the holding part 3a is coupled directly or indirectly to the base member 4. Thus, it can be said that the holding part 3a illustrated in FIG. 7A to FIG. 7C is indirectly included in the base member 4.

Note that, in the example illustrated in FIG. 7A to FIG. 7C, the holding part 3a protrudes outside the base member 4. In the surgical assistance device 1a, the diameter of the base member 4 when used for thoracoscopic surgeries may be about 30 mm to 150 mm, preferably, about 40 mm to 100 mm. Further, when used for laparoscopic surgeries, the diameter of the base member 4 may be about 30 mm to 150 mm, preferably, about 40 mm to 100 mm and may be larger than the numerical values noted above because of the absence of ribs. In the example illustrated in FIG. 7A to FIG. 7C, the diameter of the circumscribed circle of the holding part 3a can be replaced with the diameter of the base member 4 of the surgical assistance device 1a. Further, when the circumscribed shape of the holding part 3a is not circular, the shortest distance among distances obtained when the circumscribed shape is pinched by parallel lines can be defined as the diameter. Further, when the shortest distance among the obtained distances is defined as 1, the longest distance may be 2 or less, 1.9 or less, 1.8 or less, 1.7 or less, 1.6 or less, 1.5 or less, or the like.

According to the surgical assistance device 1c equipped with the holding part 3a of modified example 2, in addition to the advantageous effects achieved by the surgical assistance device 1a, an advantageous effect of less pain for the patient is achieved because the abduction in flexion of ribs can be reduced.

### [Modified Example 3 of Holding Part 3]

Fig. 8 illustrates modified example 3 of the holding part 3. FIG. 8A is a schematic top view of a surgical assistance device 1d, FIG. 8B is a sectional view taken along X-X' of FIG. 8A, and FIG. 8C is a diagram after the holding part 3a of FIG. 8B has been rotated in the R1 direction. The holding part 3a of the surgical assistance device 1d illustrated in FIG. 8A to FIG. 8C is formed rotatably relative to the base member 4, in other words, differs from modified example 2 of the holding part 3 in that the angle of the image capturing unit 2 relative to the reference line is formed to be variable.

In the example illustrated in FIG. 8A to FIG. 8C, a substantially spherical protruding part 3d is formed to the side face of the holding part base member 3b. Further, a substantially spherical recessed part 3e is formed in the base member 4. The substantially spherical protruding part 3d and the substantially spherical recessed part 3e are engaged with each other, and thereby the holding part 3a can be rotated relative to the base member 4. Further, a stopper 3f to restrict the rotation may be provided where necessary. In the example illustrated in FIG. 8B, an example in which the image capturing unit 2 is inclined relative to the reference line as much as possible is illustrated. Thus, to prevent the image capturing unit 2 from being further rotated outward, the stopper 3f is arranged to the upper part of the holding part base member 3b so as to come into contact with the base member 4. Alternatively, when the image capturing unit 2 is formed to be substantially aligned with the reference line, the stopper 3f can be provided to the lower part of the holding part base member 3b in order to allow rotation in the opposite direction to R1.

Note that the example illustrated in FIG. 8A to FIG. 8C is a mere example in which the holding part 3a is formed rotatably relative to the base member 4, and another example may be employed. For example, a substantially spherical protruding part may be formed to the base member 4, and a substantially spherical recessed part may be formed in the holding part base member 3b. Further, although depiction is omitted, a ratchet mechanism may be used so that the holding part 3a can be rotated stepwise relative to the base member 4. Further, although all the holding parts 3a are formed to be rotatable relative to the base member 4 in the example illustrated in FIG. 8A to FIG. 8C, some of the holding parts 3a may be formed to be rotatable relative to the base member 4. Further, although depiction is omitted, a cutout may be formed in the outer circumferential face of the holding part base member 3b, and the image capturing unit 2 may be fitted from the outer circumferential face of the holding part base member 3b, as illustrated in modified example 1 of the holding part 3.

According to the surgical assistance device 1d equipped with the holding part 3a of modified example 3, in addition to the advantageous effects achieved in the surgical assistance device 1a, an advantageous effect of the variable angle of the image capturing unit 2 relative to the reference line in accordance with a patient size or a use situation is achieved.

### [Modified Example 1 of Base Member 4]

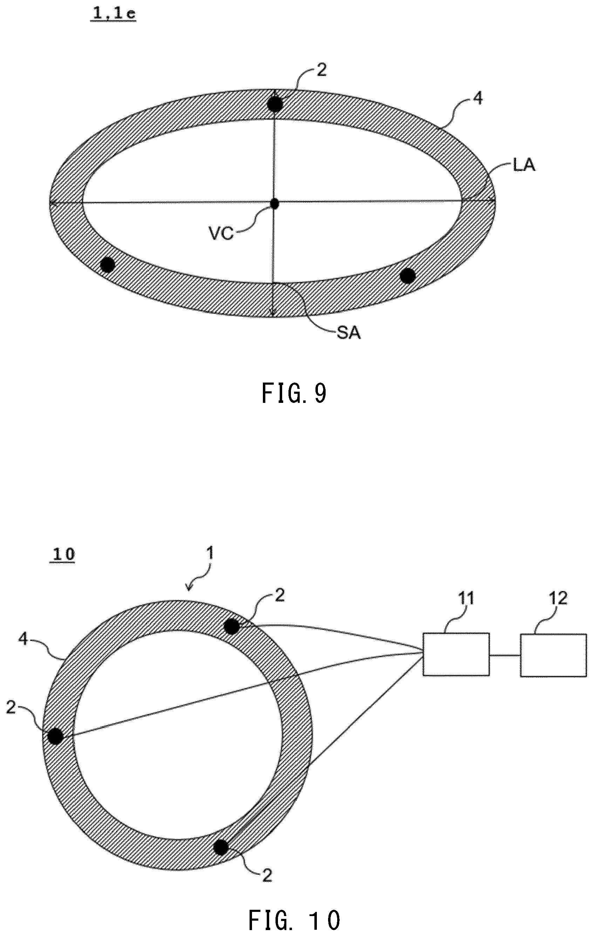

FIG. 9 illustrates modified example 1 of the base member 4. FIG. 9 is a schematic top view of a surgical assistance device 1e. In the surgical assistance device 1a according to the embodiment, the base member 4 can be made of any of a flexible material or an inflexible material. In contrast, modified example 1 illustrated in FIG. 9 differs from the surgical assistance device 1a according to the embodiment in that the base member 4 is formed of an inflexible member and formed in a substantially elliptical shape.

The ratio of the longer axis LA and the shorter axis SA of the ellipse is not particularly limited as long as it is within a range that can ensure a sufficient field of view. For example, LA/SA may be 2 or less, 1.9 or less, 1.8 or less, 1.7 or less, 1.6 or less, 1.5 or less, or the like. Note that the size of the ellipse is such that the shorter axis SA corresponds to the diameter of the surgical assistance device 1a. Further, the relationship between the image capturing unit 2 and the holding part 3 when the base member 4 is elliptical is the same as that in the surgical assistance device 1a according to the embodiment and its modified examples 1b to 1d except that the virtual center VC is the intersecting point of the longer axis LA and the shorter axis SA.

When used for a thoracoscopic surgery, the surgical assistance device 1 is required to be arranged between ribs. Thus, while the spacing between ribs can be expanded by using an instrument, the size of the surgical assistance device 1 depends on the spacing between the ribs. When the base member of the surgical assistance device 1 is substantially circular, the image capturing units are required to be arranged in the circular base member in the size described above. In contrast, in the example illustrated in FIG. 9, although the shorter axis SA depends on the spacing between ribs, the longer axis LA does not depend on the spacing between the ribs. Therefore, according to the surgical assistance device 1e equipped with the substantially elliptical base member 4 illustrated in FIG. 9, an advantageous effect of improved flexibility of the attachment position of the image capturing units 2 is achieved in addition to the advantageous effects achieved by the surgical assistance device 1a. Further, since the abduction in flexion of ribs can be reduced, an advantageous effect of less pain for the patient is also achieved.

### [Other Modified Examples]

The surgical assistance devices 1a to 1e illustrated in FIG. 1 to FIG. 9 are novel instruments that differ from instruments conventionally used for surgeries. Alternatively, a surgical assistance device may be produced by forming the holding parts 3 in the instruments already used for surgeries and attaching the image capturing units 2 thereto. For example, as an instrument used in a surgery operation, Alexis (registered trademark) wound retractor (Wound Protector) is known intended to reduce risk of wound infection, provide a good field of view, and provide the largest opening with the smallest incision size. There are various sizes of known wound retractors from an extra extra small (XXS) size for an incision length of 10 mm to 30 mm to an extra extra large (XXL) size for an incision length of 170 mm to 250 mm. A wound retractor is formed of an outer ring, an inner ring, and a film called would sheath connecting both the rings. The surgical assistance device can be produced by forming the holding parts 3 in the inner ring inserted in an incision site and attaching the image capturing units 2 thereto.

### [Embodiment of Surgery Support System]

The surgery support system 10 according to the embodiment will be described with reference to FIG. 10. FIG. 10 is a schematic diagram illustrating the overview of the surgery support system 10. The surgery support system 10 includes at least the surgical assistance device 1 and an image processing unit 11 that synthesizes images obtained from the image capturing units 2. Further, the surgery support system 10 may optionally, additionally include a display unit 12 that displays an image processed by the image processing unit 11.

As the surgical assistance device 1, any of the surgical assistance device and its modified examples (1a to 1e) illustrated in FIG. 1 to FIG. 9 may be used. The image processing unit 11 is not particularly limited as long as it can synthesize images obtained from N image capturing units 2, and such images can be synthesized by a known image synthesis method. Alternatively, an image synthesis algorithm can be produced based on a known image synthesis technology.

The display unit 12 is not particularly limited as long as it can display an image processed by the image processing unit 11, and a known monitor such as a liquid crystal monitor, an organic EL monitor, or the like can be used.

### [Embodiment of Program]

The image processing unit 11 stores a program that can synthesize images obtained from N image capturing units 2. The program is not particularly limited as long as it can synthesize images obtained from N image capturing units 2 and can be used as a program of the surgery support system.

The program may perform image processing using the feature of the surgical assistance device 1 disclosed in the present application. For example, since the surgical assistance device 1 disclosed in the present application is equipped with N image capturing units 2, even when one image capturing unit 2 is unable to capture an image of a part inside a body cavity due to a surgical instrument, another image capturing unit 2 may be able to capture an image inside the body cavity. In such a case, the program may perform image processing so that, when some of the image capturing unit 2 captures an image of a surgical instrument, an image captured by another image capturing unit 2 can be displayed. Image capturing of a surgical instrument (detection of a surgical instrument) can be detected in accordance with a change in the color in a captured image, a sudden motion, or the like due to the surgical instrument.

Further, there may be a case where regions that can be captured by different image capturing units 2 do not overlap, in other words, there may be a case where an image of a surgical instrument is captured in a region captured by a single image capturing unit 2. In such a case, the program can perform image processing by using an image inside the body cavity captured before image capturing of the surgical instrument is detected. For example, images inside the body cavity may be captured and stored in advance before a surgery is started or images in the past several seconds may be stored in advance, and image processing can be performed so that the surgical instrument is not visible based on the stored images.

Further, the program may perform image processing so as to be able to display a VR-like image such as displaying information required for a surgery or the like in combination, where necessary, instead of displaying the image captured by the image capturing unit 2 as it stands. When these programs are provided as separate components, the user-friendliness of the surgery support system is improved.

Note that the disclosure in the present application is not limited to the embodiments described above. Any combination of respective embodiments described above, modification of some component of each embodiment, or omission of some component is possible within the scope of the technical concept disclosed in the present application. Furthermore, some component may be added to each embodiment described above. For example, a function of changing the frequency of a light source irradiating inside of a body cavity to make the depth inside the body cavity known may be added.

Although Examples will be provided below and the embodiment disclosed in the present application will be specifically described, the Examples are merely for illustration of the embodiment, which are intended neither to limit the scope of the invention disclosed in the present application nor to express limitation of the same.

### [EXAMPLES]

### [Production of Surgical Assistance Device]

### <Example 1>

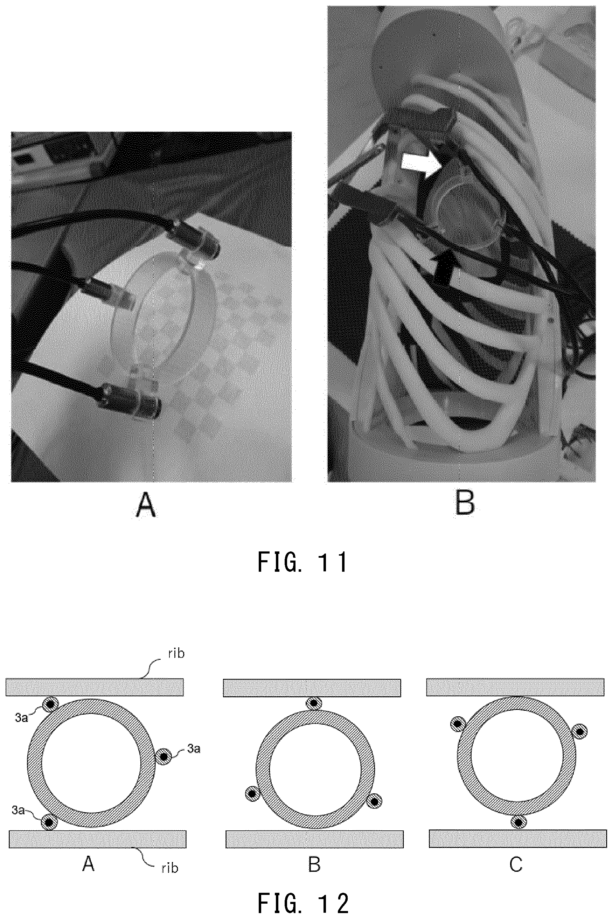

As the material forming the holding parts 3a and the base member 4, Agilista-3100 (by KEYENCE CORPORATION) was used, and a 3D printer was used to mold the holding parts 3a and the base member 4 in one piece. Three holding parts 3a were formed at equal intervals. The base member 4 was substantially cylindrical, and the diameter of the base member 4 was about 60 mm. Further, the length from the outer circumference of the base member 4 to the end of the holding part 3a (the length of a protruding portion from the base member 4) was about 17 mm. Next, MD-3110VL-NU-158 (Weld Vision Limited Liability Company) was used as the image capturing units 2 and inserted in the holding parts 3a to produce the surgical assistance device 1. FIG. 11A is a photograph of the surgical assistance device produced in Example 1. Note that respective angles of three image capturing units 2 relative to the reference line were designed to be the same. Further, to implement various Examples described later, surgical assistance devices were produced in which each angle of the image capturing units 2 relative to the reference line was 0 degree, 3 degrees, 5 degrees, and 10 degrees, respectively. Note that the angle of the image capturing unit 2 relative to the reference line was designed so as to be oriented to outside from the center of the base member 4.

### [Production of Surgery Support System]

### <Example 2>

The image capturing units of each surgical assistance device produced in Example 1 was connected to the image processing unit that synthesizes images by using an algorithm developed by the inventor (the function was expanded based on Open Source Computer Vision Library (OpenCV). Next, the image processing unit was connected to the monitor to produce the surgery support system.

### [Observation inside Body Cavity Using Mock Human Body]

Next, the surgical assistance device of the surgery support system produced in Example 2 was inserted between ribs of a mock human body. FIG. 11B is a photograph after the surgical assistance device was inserted in the mock human body. In FIG. 11B, the black arrow portion indicates the inserted surgical assistance device. Further, the mock human body illustrated in FIG. 11B is Fasotec THORA (by FASOTEC Co., Ltd.), and the white arrow portion indicates a mock lung.

FIG. 12 is a diagram illustrating the arrangement of the surgical assistance device in an observation experiment inside the body cavity using a mock human body. In the example illustrated in FIG. 12A, the surgical assistance device was arranged such that the line connecting two holding parts 3a is substantially perpendicular to the upper and lower ribs and the remaining holding part 3a is located at the center between the ribs. In the example illustrated in FIG. 12B, the surgical assistance device was arranged turned by 30 degrees clockwise from the arrangement illustrated in FIG. 12A. In the example illustrated in FIG. 12C, the surgical assistance device was arranged turned by 30 degrees counterclockwise from the arrangement illustrated in FIG. 12A. The images A, B, C of each drawing illustrated in the following Examples are images captured at the positions A, B, C of FIG. 12, respectively.

### <Example 3 to Example 7>

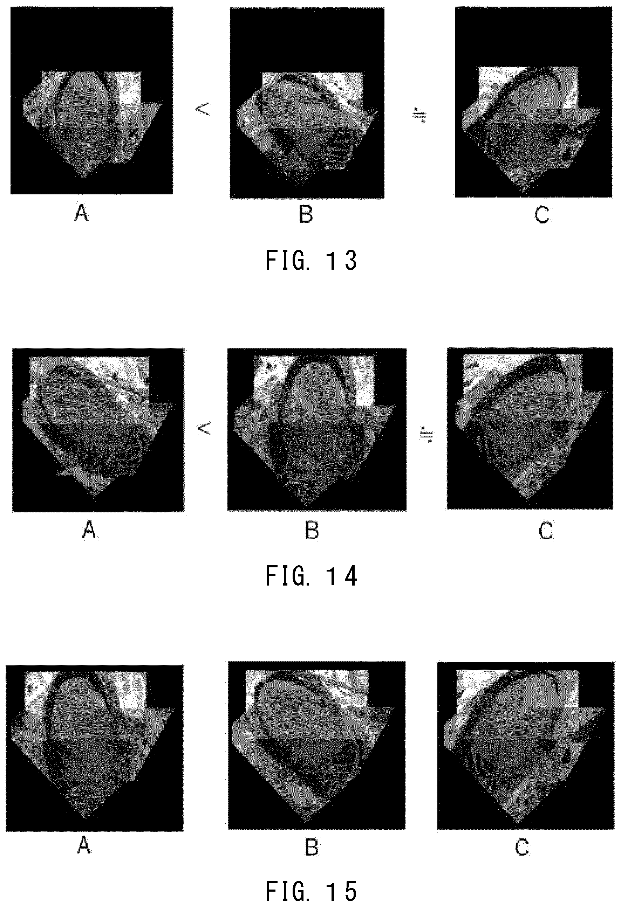

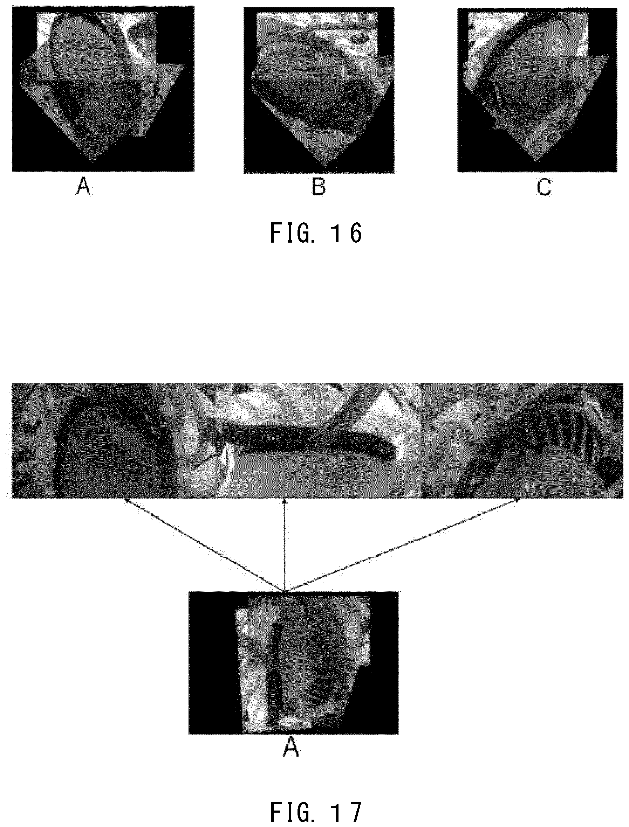

The angle of the image capturing unit 2 relative to the reference line was:
- 0 degree (Example 3) in images indicated in FIG. 13;
- 1 degree (Example 4) in images indicated in FIG. 14;
- 3 degrees (Example 5) in images indicated in FIG. 15;
- 5 degrees (Example 6) in images indicated in FIG. 16; and
- 10 degrees (Example 7) in images indicated in FIG. 17.

Although a partial loss was found in the upper part of the mock lung in the synthesized image illustrated in FIG 13A, a sufficient field of view was ensured inside the thoracic cavity including the mock lung in the synthesized images illustrated in FIG. 13B and FIG. 13C. Therefore, it was confirmed that, even when the surgical assistance device having the angle of 0 degree produced in Example 1 is used, a field of view required for an arthroscopic surgery can be ensured with adjustment of the arrangement of the image capturing units relative to the ribs.

The synthesized images illustrated in FIG. 14A to FIG. 14C all have a wider area of the field of view than those in Example 3 in which the image capturing unit 2 has the angle of 0 degree. Further, the partial loss in the upper part of the mock lung found in the synthesized image of FIG. 13A is not found in FIG. 14A, and thus the whole mock lung was able to be viewed.

In the synthesized images illustrated in FIG. 15A to FIG. 15C and FIG. 16A to FIG. 16C, it was confirmed that a larger angle of the image capturing unit 2 results in a larger area of the field of view.

In contrast, in the synthesized image illustrated in FIG. 17A, although the area of the field of view was further increased, the center of the mock lung was slightly unclear. Thus, review was made on images captured by respective image capturing units before image synthesis. Three images on the upper part in FIG. 17A are images captured by respective image capturing units before image synthesis. From the three images in the upper part, it was found that each image capturing unit more captures an image of the portion outside the mock lung and less captures an overlapping image of the center of the mock lung. There is no problem with the case where the angle is 10 degrees illustrated in FIG. 17 in terms of ensuring a sufficient field of view during a surgery. It was confirmed that it is desirable to set the angle at about 10 degrees additionally in terms of observation of tissues inside the thoracic cavity.

### Comparative Example 1>

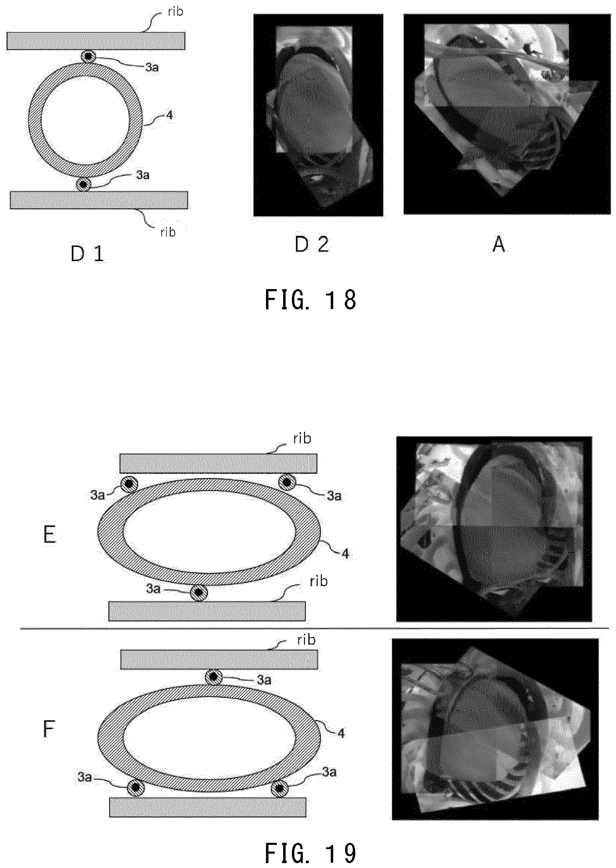

FIG. 18D1 illustrates a surgical assistance device produced in Comparative example 1 and the arrangement thereof relative to a mock human body. In the surgical assistance device of Comparative example 1, the number of holding parts 3a holding image capturing units was two, which were arranged such that the line connecting two holding parts 3a passed through the substantial center of the base member 4. The angle of the image capturing unit relative to the reference line was 1 degree, and the two holding parts 3a were arranged in contact with ribs. FIG. 18D2 is a synthesized image obtained in Comparative example 1. Further, for comparison, the synthesized image for the arrangement A of Example 4 (FIG. 14A) is juxtaposed and illustrated in which the angle of the image capturing unit is the same 1 degree and the two holding parts 3a are arranged near the ribs. As is clear from the comparison between FIG. 18D2 and FIG. 18A, a partial loss of the mock lung was found in the case of two image capturing units. It was therefore confirmed that the number of image capturing units is required to be two or greater.

### <Example 8>

FIG. 19E and FIG. 19F illustrate a surgical assistance device produced in Example 8 and the arrangement thereof relative to a mock human body. The surgical assistance device produced in Example 8 was produced in the same procedure as that in Example 1 except that:
- the base member 4 was elliptical having the longer axis of 6 cm and the shorter axis of 3 cm;
- one of the holding parts 3a was arranged at the intersecting point of the shorter axis of the ellipse and the base member, and the remaining two are arranged at equal intervals so as to form an angle of 120 degrees with respect to the ellipse center, respectively; and
- the angle of each image capturing unit is 5 degrees, and each image capturing unit is oriented to outside from the ellipse center. Next, the surgery support system was produced in the same procedure as that in Example 2.

The right side in FIG. 19E and FIG. 19F are synthesized images in accordance with the arrangement of the surgical assistance device indicated on the left side. As illustrated in FIG. 19E and FIG. 19F, it was confirmed that image capturing can be performed without any problem even when the base member 4 is elliptical.

### [Industrial Applicability]

The surgical assistance device and the surgery support system disclosed in the present application can ensure a wide field of view during an arthroscopic surgery, which are therefore useful for manufacturing industries for medical devices.

### [List of References]

- 1, 1a to 1e: surgical assistance device
- 2: image capturing unit
- 3, 3a: holding part
- 3b: holding part base member
- 3c: through hole
- 3d: spherical protruding part
- 3e: spherical recessed part
- 3f: stopper
- 31: upper end of a holding part
- 32: lower end of a holding part
- 4: base member
- 10: surgery support system
- 11: image processing unit
- 12: display unit
- BL: reference line
- CL: center line of an image capturing unit
- dα: direction of angle α
- IL: image capturing range
- LA: longer axis
- SA: shorter axis
- VC: virtual center
- VF: virtual plane
- VL: virtual line

## Claims

1. A surgical assistance device for capturing an image inside a body cavity, the surgical assistance device comprising:
N image capturing units;
holding parts, each of the holding parts holding each of the image capturing units; and
a base member,
wherein N is an integer that is greater than or equal to three, and
wherein when N virtual lines each passing through each of the holding parts holding the N image capturing units are defined as reference lines for arrangement when the N virtual lines are
substantially parallel to each other, and
substantially perpendicular to a virtual plane formed by the base member or the holding parts,
each of the image capturing units is arranged so as to be oriented to outside at an angle that is greater than or equal to 0 degree and less than or equal to 10 degrees relative to each of the reference lines passing through each of the holding parts.

2. The surgical assistance device according to claim 1, wherein the holding parts are formed as separate components from the base member.

3. The surgical assistance device according to claim 1 or 2, wherein the angle of at least one of the N image capturing units is variable.

4. The surgical assistance device according to claim 2 or 3, wherein the holding parts formed as the separate components from the base member are formed rotatably relative to the base member.

5. The surgical assistance device according to any one of claims 1 to 4, wherein the base member is formed of a flexible material.

6. The surgical assistance device according to any one of claims 1 to 5, wherein each of the image capturing units is arranged so as to be oriented to outside of the base member at an angle that is greater than or equal to 5 degrees and less than or equal to 10 degrees relative to each of the reference lines passing through each of the holding parts.

7. A surgery support system comprising:
the surgical assistance device according to any one of claims 1 to 6; and
an image processing unit that synthesizes images obtained from the image capturing units.

8. The surgery support system according to claim 7 further comprising a display unit that displays an image processed by the image processing unit.

9. A program used for the surgery support system according to claim 8.

10. The program according to claim 9, wherein when any of the image capturing units captures a surgical instrument during a surgery, the program performs image processing so that the surgical instrument is not displayed on the display unit based on
an image captured by another of the image capturing units, or
an image inside a body cavity captured before image capturing of the surgical instrument is detected.
